# EUROPEAN PATENT APPLICATION

(11) **EP 2 477 028 A1**
(43) Date of publication of application: **18.07.2012**
(21) Application number: 12151363.4
(22) Date of filing: 17.01.2012
(51) Int. Cl.: G01N 33/487, G01N 27/327, G01N 27/447

(54) **Analysis device, program and method with calibration based on test device identification**

(30) Priority: 18.01.2011 JP 2011008318
(71) Applicant: ARKRAY, Inc., Kyoto-shi, Kyoto 601-8045 (JP)
(72) Inventor: Sugiyama, Koji, Kyoto, 602-0008 (JP); Shiraki, Yasunori, Kyoto, 602-0008 (JP)
(74) Representative: HOFFMANN EITLE

(57) **Abstract**

An analysis device (1) comprises an identification data acquisition unit (13) for acquiring an identifier of a mounted measurement device (9), a calibration unit (11) for recording calibration data of the measurement device associated with the identifier of the measurement device (9) to a recording device (3), and a computing unit (12) that calculates a value indicating the information related to the measurement target component by using measurement data obtained by detection of the measurement target component by a detector and calibration data that has been recorded to the recording device (3) associated with the identifier of the measurement device acquired by the identification data acquisition unit (13).

## Description

### FIELD OF THE INVENTION

The present invention relates to an analysis device and an analysis program for using a removable measurement device to analyze components contained in a sample.

### BACKGROUND OF THE INTENTION

With an analysis device that makes use of a separating analysis method, a separation device is used for separating a measurement target component in a sample. For example, in measurement using capillary electrophoresis, a capillary is used as the separation device. The sample is introduced into the capillary, and voltage is applied in the channel direction to separate the components in the sample.

With a device that makes use of liquid chromatography, a column filled with a packing agent is used as the separation device. The sample is introduced into the column, and fractions are eluted for the various components. With a device in which capillary electrochromatography is used, a capillary filled with a packing agent is used as the separation device. The sample is introduced into the capillary, and voltage is applied in the channel direction to separate the components in the sample.

With an analysis device that makes use of one of these separating analysis methods, the separation device is usually replaceable. For example, a technique has been developed in which the capillary used in capillary electrochromatography is made into a microchip (see Non-Patent Document 1 and Patent Document 1, for example). This microchip is easy to replace. Examples of the application of a separating analysis method in the field of clinical testing include HbA1c measurement devices, glycoalbumin measurement devices, and so forth (see Non-Patent Document 2, for example). An HPLC (high performance liquid chromatography) method featuring a column, for example, is used with these devices. Also, even with an analysis device that does not make use of a separating analysis method, it may be that the measurement device that holds the sample can be replaced.

### PRIOR ART DOCUMENT

Patent Document 1: Japanese Laid-open Patent Publication No. 2006-189401A

Non-Patent Document 1: Arai Akihiro et al., "Development of Microchip Electrophoresis Apparatus MCE 2010 and its Application, Shimadzu Hyoron, Vol. 58, No. 3/4, pp. 101-109, March 2003.

Non-Patent Document 2: "Basic Research into HbA1c with Adams A1c HA-8170," Igaku to Yakugaku, Vol. 58, No. 2, pp. 355-361, 2007.

### SUMMARY

The inventors realized that in analysis using an interchangeable measurement device, calibration (correction) of the analysis device is required in every time the measurement device is replaced. Specifically, the calibration result (such as a correction coefficient) is often decided by the combination of a specific measurement device and a specific analysis device (such as a control device or electrophoresis device). Accordingly, in a situation in which the measurement device is replaced frequently, it will be necessary to perform calibration at every replacement, which entails more work. For example, a measurement device that has been made into a microchip can be easily replaced, which is its most salient feature. However, because of the extra work of performing calibration at every replacement, the convenience resulting from the ease of replacing the measurement device ends up being cancelled out to a certain extent. This happens even with an analysis device that does not make use of a separating analysis method, and makes use of some other interchangeable measurement device.

In view of this, it is an object of the present invention to reduce the work entailed by calibration when replacing the measurement device, an example of which is a measurement device.

The analysis device according to one embodiment is an analysis device that controls a detector that detects a measurement target component in a sample held by a removable measurement device for holding the sample in a state that allows measurement, and the measurement device, and thereby acquires measurements data and analyzes the measurement target component. The analysis device includes an identification data acquisition unit adapted to acquire an identifier of the measurement device mounted to the analysis device, a calibration unit adapted to record calibration data associated with the identifier of the measurement device to at least one of recording device provided to the analysis device or recording device that is accessible from the analysis device, and a computing unit that calculates a value indicating information related to the measurement target component by using measurement data obtained by detection of the measurement target component by the detector and calibration data that has been recorded to the recording device associated with the identifier of the measurement device acquired by the identification data acquisition unit.

In one exemplary embodiment of the present disclosure, an identifier is acquired for a mounted measurement device, and calibration data obtained by, for example, using this measurement device to measure a calibration sample is associated with the identifier of the measurement device and recorded. Accordingly, once calibration is performed with the measurement device mounted to the analysis device, and the recorded calibration data can be used in measurement performed using this measurement device. As a result, there is no need to perform calibration every time the measurement device is mounted, and this reduces the amount of work entailed.

### BRIEF DESCRIPTION OF THE DRAWINGS

FIG.1 is a function block diagram showing an example of the configuration of an analysis system that includes an analysis device in a first exemplary embodiment.
FIG. 2 is a flowchart showing an example of the operation of an analysis device.
FIG. 3 is a table showing an example of the ID for a measurement device and calibration data that has been associated and recorded.
FIG. 4 is a function block diagram showing an example of the configuration of an analysis system that includes an analysis device in a second exemplary embodiment.
FIG. 5 is a table showing an example of a measurement device in which calibration data that has been recorded after being associated with the ID of an analysis device.
FIG. 6 is a function block diagram showing an example of the configuration of an analysis system that includes an analysis device in a third exemplary embodiment.
FIG. 7 is a table showing an example of data recording the number of measurements.
FIG. 8 is a flowchart showing an example of the operation of an analysis device in the third embodiment.
FIG. 9 is a table showing an example of calibration data in a fourth exemplary embodiment.
FIGS. 10A to 10C are diagrams showing an example of the configuration of a measurement device that has been made into a microchip (hereinafter referred to as an analysis chip) in a fifth exemplary embodiment.
FIG. 11 is a table showing an example of calibration data in the fifth embodiment.
FIG. 12 is an oblique view of an example of a treatment device.
FIG. 13 is an exploded oblique view of an example of an ion activity measurement tool.

### DETAILED DESCRIPTION

The analysis device according to one exemplary embodiment is an analysis device that controls a detector that detects a measurement target component in a sample held by a removable separation device for separating the measurement target component from the sample, and, and thereby acquires measurement data and analyzes the measurement target component. The analysis device includes an identification data acquisition unit for acquiring an identifier of the separation device mounted to the analysis device, a calibration unit for recording calibration data associated with the identifier of the separation device to at least one of recording device provided to the analysis device or external recording device that is accessible from the analysis device, and a computing unit that calculates a value indicating the amount of the measurement target component by using measurement data obtained by detection of the measurement target component by the detector and calibration data that has been recorded to the recording device associated with the identifier of the separation device acquired by the identification data acquisition unit.

With the above constitution, an identifier is acquired for the mounted separation device, and calibration data obtained by using this separation device to separate a calibration sample is associated with the identifier of the separation device and recorded. Accordingly once calibration is performed with the separation device mounted to the analysis device, the recorded calibration data can be used in measurement performed using this separation device. As a result, there is no need to perform calibration every time the separation device is mounted, and this reduces the amount of work entailed.

The recording device may be accessible from the analysis device. For example, the recording device may be provided to the analysis device, or may be provided to a device other than the analysis device, which is accessible from the analysis device. For example, the recording device may be provided to either the analysis device or the measurement device, or the analysis device and the measurement device may both be equipped with a recording device. The recording device may be a storing means such as memory or storage.

The calibration unit may produce or update the calibration data by using measurement data obtained by detection of the measurement target component in the calibration sample in the measurement device. Thus, the calibration data can be produced or updated by using measurement data for the calibration sample measured with the measurement device mounted to the analysis device.

As one exemplary embodiment of the analysis device, when the measurement device is mounted, the calibration unit determines whether or not calibration data associated with an identifier of the mounted measurement device has been recorded to the recording device, and if the calibration data has not been recorded to the recording device, the calibration data is produced on the basis of measurement data obtained by detection of the measurement target component in a calibration sample held by the mounted measurement device, and this calibration data is recorded to the recording device with being associated with the identifier of the mounted measurement device.

Consequently when a new measurement device is mounted, calibration is performed for that measurement device, and calibration data can be associated with an identifier of the measurement device and recorded.

As one exemplary embodiment of the analysis device, the identification data acquisition unit acquires an identifier of an analysis device, the calibration unit records the identifier of the analysis device associated with the calibration data to a recording device provided to the mounted measurement device, and the computing unit calculates a value indicating information related to the measurement target component by using measurement data obtained by detection of the measurement target component by the detector and calibration data that has been recorded to the recording device of the measurement device associated with the identifier of the analysis device acquired by the identification data acquisition unit.

Consequently, calibration data and an identifier of the calibration-executed analysis device can be associated with the measurement device and recorded. Accordingly, even when measurement devices are mounted to various analysis devices and measurements made with each, for example, the proper calibration data can be used according to the combination of measurement device and analysis device.

As one exemplary embodiment of the analysis device, when the measurement device is mounted, the calibration unit determines whether or not the calibration data associated with the identifier of the measurement device has been recorded to the recording device of the measurement device, and if the calibration data has not been recorded to the recording device, the calibration data is produced and recorded to the recording device with being associated with the identifier of the mounted measurement device.

Consequently when a new measurement device is mounted, calibration is performed using that measurement device, and calibration data and an identifier of the analysis device can be associated and recorded.

As one exemplary embodiment of the analysis device, the number of measurements of the measurement target component using the measurement device is recorded to the recording device associated with the identifier of the measurement device by the computing unit, and the computing unit can determine whether or not measurement can be performed using said measurement device based on the number of measurements.

Consequently, the number of times the measurement device is used for measurement can be properly controlled.

As one exemplary embodiment of the analysis device, the computing unit records the elapsed time and/or the number of measurements after the previous calibration of the measurement device associated with the identifier of the measurement device to the recording device, and the calibration unit determines whether or not to perform calibration based on the elapsed time and/or the number of measurements after the previous calibration.

Consequently, it is possible to perform the proper calibration after the desired number of measurement specimens or the desired length of time.

As one exemplary embodiment of the analysis device, the calibration unit also records the identifier of the analysis device associated with the calibration data and the identifier of the measurement device, and when the measurement device has been mounted, the calibration unit determines whether or not calibration data associated with the identifier of the mounted measurement device and the identifier of the analysis device has been recorded to the recording device, and if the calibration data has not be recorded to the recording device, the calibration data is produced and recorded to the recording device with being associated with the identifier of the mounted measurement device and the identifier of the analysis device. Consequently, and timing and frequency of calibration of the measurement device can be properly controlled.

As one exemplary embodiment of the above analysis device, the measurement device comprises a plurality of channels for separating a sample, the identifier of the measurement device includes an identifier of each channel that the measurement device comprises, the calibration unit produces or updates the calibration data for each of the plurality of channels, and associates and records the channel identifiers with the calibration data, and the computing unit calculates a value indicating information related to the measurement target component by using the measurement data obtained by detection of one channel of the measurement target component by the detector and calibration data that have been recorded to the recording device associated with the identifier of the channel included in the identifier of the measurement device acquired by the identification data acquisition unit.

An exemplary embodiment consistent with of the present disclosure also encompasses an analysis program that causes a computer to function as the above-mentioned analysis device, as well as a recording medium to which this program is recorded. The embodiment also encompasses an analysis method executed by the above-mentioned analysis device.

According to embodiments of the present disclosure, in measurement using a removable measurement device, the amount of work entailed by calibration when the measurement device is replaced can be reduced.

Here, "an information" related to the measurement target component may contain all information obtained as a result of an analysis for the measurement target component. Examples thereof contains "amount", "site", "weight", "optical characteristics" etc. of the measurement target component without limitation.

### First Embodiment

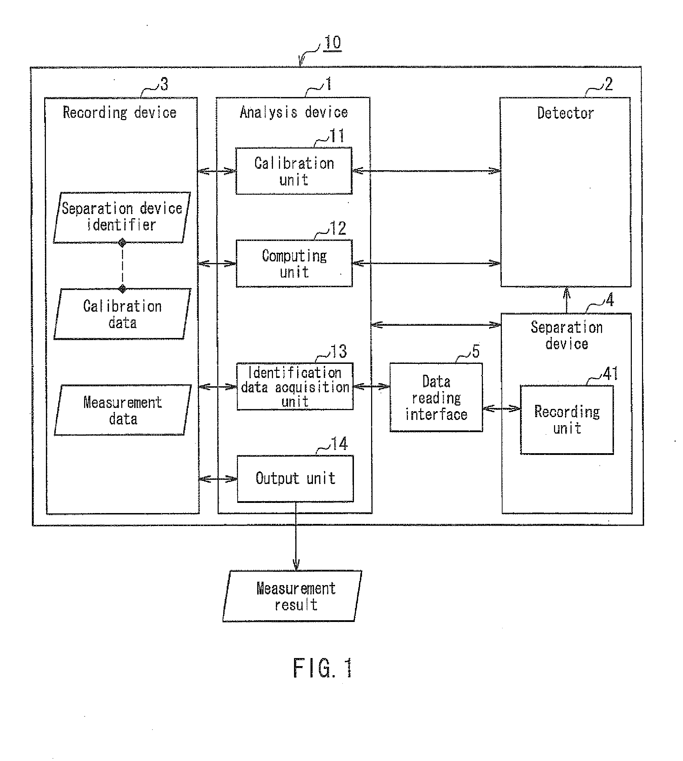

FIG. 1 is a function block diagram showing an example of the configuration of an analysis system that includes an analysis device in a first exemplary embodiment. The analysis system 10 shown in FIG. 1 is an analysis system that makes use of a separating analysis method, in which the components contained in a sample are separated, and the separated components are quantified. An analysis device 1 is connected to a detector 2, a separation device 4, and a recording device 3. The separation device 4 is used to separate a measurement target component from a sample, and can be attached to and removed from the analysis device 1. The detector 2 detects the measurement target component separated by the separation device 4. The analysis device 1 controls the detector 2, acquires measurement data, and analyzes the measurement target component in the sample. Here, the analysis device 1 can also control the separation device 4. However as for this embodiment, the analysis device may control only a detector or may control both of a detector and a separation device (a measurement device).

The term "separating analysis method" in this Specification refers to a method in which analysis is performed while analytes contained in a sample are individually separated. Examples include liquid chromatography (HPLC), capillary electrophoresis (CE), and capillary electrochromatography. Examples of liquid chromatography include cation exchange chromatography, anion exchange chromatography, partition chromatography, reverse phase partition chromatography, gel filtration chromatography, and affinity chromatography. Examples of capillary electrophoresis include capillary zone electrophoresis, capillary isotachophoresis, capillary isoelectrophoresis, capillary electrokinetic chromatography, and capillary gel electrophoresis.

A separation device is an example of a measurement device that holds a sample in a state that allows measurement. The separation device is used to separate measurement target components in a sample. For example, a device that includes a column as a separation device is used in liquid chromatography. In this case, although not shown in the drawings, for example, a liquid supply unit for supplying an eluate, cleaning liquid and the like to the column, a sample supply unit for supplying a sample to the column, a valve or other such adjusting mechanism for adjusting the flow of sample and eluate, or another such necessary member may be provided along with the column.

With capillary electrophoresis, a device that includes a capillary is used as the separation device. In this case, although not shown in the drawings, for example, a high-voltage power supply, a specimen supply unit, or another necessary member may be provided along with the separation device. Also, a capillary, an electrode for applying voltage to the capillary, and so fourth are provided to the separation device.

With capillary electrokinetic chromatography, a device that includes a capillary filled with a packing agent is used as the separation device. In this case, although not shown in the drawings, for example, a high-voltage power supply, a specimen supply unit, or another necessary member may be provided along with the separation device. Also, a capillary, an electrode for applying voltage to the capillary and so forth are provided to the separation device.

The separation device may also be made into a microchip. This makes it easier to replace the separation device. Accordingly, a plurality of separation devices for different measurement applications canbe frequently exchanged for measurement, or a separation device that is a delicate device chip can be removed from the analysis device (such as an electrophoresis device) and stored.

The detector 2 detects the absorbance of the sample separated by the separation device, for example, and can detect the various components in the sample. For example, the separated components in the sample can be detected using transmissivity, fluorescence, or another such method.

The measurement data obtained by detecting the measurement target component separated from the sample by the detector 2 may be, for example, data (such as a chromatogram or a pherogram) indicating the time change or position change (distribution) of a signal strength obtained by detecting the component separated from a sample, or may be a value related to the separation state of a component obtained from this data. More specifically, at least one of time series data or waveform data about the detected quantity for the component separated from a sample, the time it takes for the detected quantity for the component to reach its peak, the full width at half maximum, the peak surface area (including the surface area ratio of the peak of the component with respect to the peak surface area of the total components), the signal strength of the peak, or the bottom time for the peak, for example, can be obtained as measurement data for the detector 2. Thus, the measurement data may be data expressing the separation result obtained by separating the measurement target component from the sample. The measurement data is not limited to any particular form. Furthermore, it may be the configuration in which the measurement data is outputted by the detector 2, or it may be the configuration in which the measurement data is produced on the basis of the signal detected by the detector 2, outside of the detector 2 (such as by the analysis device 1).

The recording device 3 may be any recording medium that can be accessed by the analysis device 1. For example, the recording device 3 may be a memory provided internally to the analysis device 1, or may be an external recording device. Alternatively, a recording device that is internal to the analysis device and an external recording device can both be used. More specifically, the recording device 3 can be an EEPROM or the like.

The analysis device 1 comprises a calibration unit 11, a computing unit 12, an identification data acquisition unit 13, and an output unit 14.

The identification data acquisition unit 13 acquires an identifier of the separation device mounted to the analysis device 1. In this embodiment, the identification data acquisition unit 13 acquires an identifier (hereinafter referred to as an ID) for the separation device 4 from a recording unit 41 of the separation device 4 mounted to the analysis device 1, via a data reading interface 5. The identifier of the separation device 4 is an ID unique to that separation device. There are no particular restrictions on the form of the data reading interface 5 or the recording unit 41. The recording unit 41 can be an electronic chip (memory, etc.), printing (barcode, etc.), engraving, an electronic switch, a mechanical switch, or the like. The recording unit 41 is an example of a recording device that is accessible from the analysis device 1. The data reading interface 5 may be a reader that reads printing if the recording unit 41 is printing, and may be a memory controller if the recording unit 41 is an electronic chip (memory).

The calibration unit 11 sends a control signal to the detector 2 causing the detector 2 to detect a separated measurement target component separated from a calibration sample in the separation device 4, which allows measurement data for the calibration sample to be acquired. The calibration unit 11 produces or updates calibration data by using the measurement data for the calibration sample and records the calibration data associated with the ID of the separation device 4 to the recording device 3. A sample in which an amount of a measurement target component is known can be used as the calibration sample. Consequently, a relation is obtained between the detected amount of the measurement target component in the calibration sample separated by the separation device and the amount of the measurement target component in the calibration sample. Data representing this relation can be used as calibration data.

The calibration data is used to correct the detected value produced from the separation device, so almost all the calibration data is unique to each separation device. For example, the calibration data can be used to convert the detected amount of measurement target component detected by the detector 2 into a value indicating information related to the measurement target component in the sample. More specifically, a formula or table giving a correspondence relation between the value of the detected amount of the detector 2 and the value of the amount of measurement target component can be recorded to the recording device 3 as calibration data. The value indicating the amount of measurement target component may be the value of a relative amount or of an absolute amount. The calibration data is not limited to the data showing a relation between detected amount and amount of the measurement target component. The calibration data may be data showing a relation between a detected value and a value (such as value showing feature of the target component obtained as a result of the analysis) indicating information related to the target component.

The computing unit 12 uses measurements data about the sample obtained by detection with the detector 2 and calibration data recorded to the recording device 3 to calculate a value indicating the amount of the measurement target component. The computing unit 12 here can read from the recording device 3 calibration data associated with the ID of the separation device acquired by the identification data acquisition unit 13, and use the calibration data to calculate the value indicating the amount of the measurement target component. Consequently, calibration data that is suited to the mounted separation device 4 can be used.

The output unit 14 outputs to the user a quantification value calculated by the computing unit 12. For example, this quantification value may be displayed on a display (not shown) provided to the analysis device 1, or can be outputted to a display, printer, speaker, or other such output device connected to the analysis device 1, or outputted to another computer connected via a network.

The functions of the analysis device 1 shown in FIG. 1 may be realized by executing a specific program with a microprocessor built into a measurement device or a general purpose computer equipped with a CPU, such as a personal computer, for example. That is, the analysis device 1 may be constituted by a computer capable of operating in conjunction with the detector 2 or the separation device 4. Data communication between the separation device 4 and the analysis device 1, and data communication between the analysis device 1 and the detector 2 may be carried out by wire or wirelessly.

The recording device 3 may be a memory, a hard disk drive, or another such recording device that is accessible from the processor of a computer. The analysis system 10 may have a configuration in which the analysis device 1, the detector 2, the separation device 4, and the recording device 3 are integrated, or may be such that a general purpose computer is connected to the detector 2 and the separation device 4. Also, program for causing a computer to function as the analysis device 1, or a recording medium to which this program is recorded, is also encompassed by embodiments of the disclosure. An analysis method executed by a computer is also an aspect of the present disclosure. Here, the term "recording medium" does not include non-transitory media, such as a signal itself.

### Operation Example

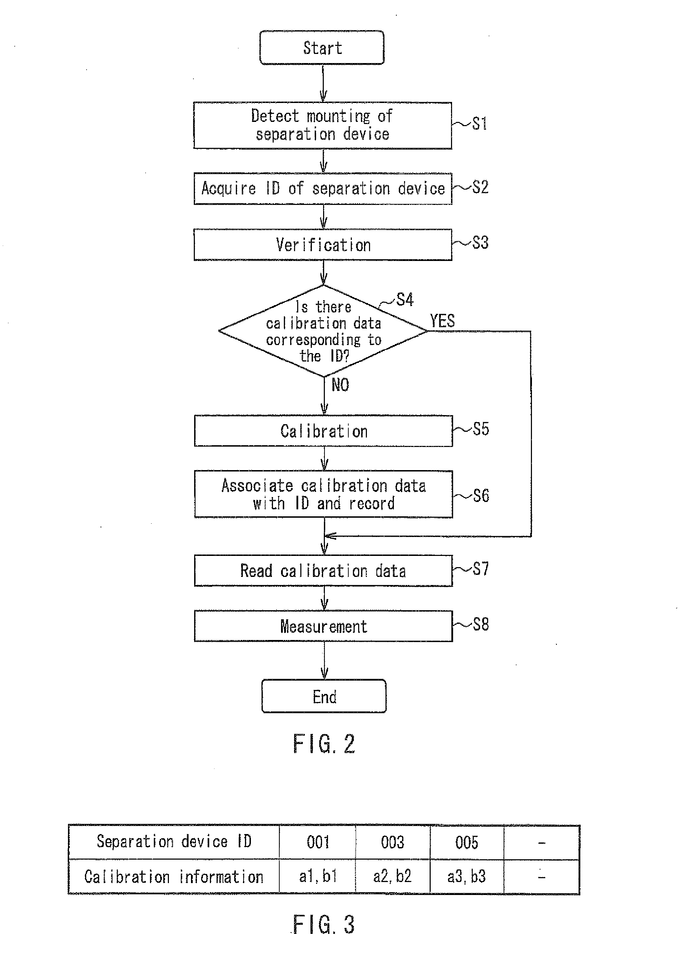

FIG. 2 is a flowchart showing an example of the operation of the analysis device 1. In the example shown in FIG. 2, a separation device mounted to the analysis device 1 is detected (S1). For example, the mounting of a separation device can be detected by detecting mechanical or electrical contact, but there are no particular restrictions on the detection method. Also, the separation device may be configured such that a plurality of them can be mounted at the same time. If plural separation devices are mounted, the processing for selecting the separation device to be the object of measurement may be the detection processing in step S1.

The identification data acquisition unit 13 acquires the ID of the mounted separation device 4 (S2). For example, if the data reading interface 5 is a barcode reader, and the recording unit 41 of the separation device 4 is a barcode, the identification data acquisition unit 13 controls the barcode reader to read the barcode of the mounted separation device 4, allowing information about the read barcode to be acquired.

The calibration unit 11. refers to the recording device 3 and determines whether or not calibration data corresponding to the ID of the separation device 4 acquired by the identification data acquisition unit 13 has been recorded (S3). FIG. 3 is a diagram showing an example of the ID for the separation device and calibration data that has been associated and recorded to the recording device 3. In the example shown in FIG. 3, calibration information (coefficients a and b of a formula indicating a calibration curve are given here as an example) is recorded for each separation device ID. For example, the calibration unit 11 can determine in S4 whether or not the separation device ID acquired in S2 is present in the data shown in FIG. 3. This determination of S4 allows a determination as to whether or not the mounted separation device 4 has been mounted for the first time to the analysis device 1.

If there is no calibration data corresponding to the ID of the mounted separation device in the recording device 3 (i.e., No in S4), the analysis device 1 executes calibration (S5). This calibration may be executed, for example, by outputting a message to an operator recommending calibration, or automatic calibration processing may be executed. This automatic calibration processing can be executed, for example, by sending a control signal to the separation device 4 or another drive means, so that a calibration sample is separated by the separation device 4 and the measurement target component thus separated is detected by the detector 2. The calibration data is produced on the basis of measurement data obtained by detecting the measurement target component in the calibration sample.

For example, the calibration unit 11 can obtain a pherogram or chromatogram of the calibration sample as the measurement data, use this to calculate a calibration curve, and record the calibration curve as calibration data to the recording device 3. When measuring HbA1c, for example, a calibration curve can be produced that shows the corresponding relation between the HbA1c measurement result for a calibration sample obtained from a pherogram or chromatogram (this is equal to the surface area of the HbA1c fraction divided by the surface area of the total Hb fraction, times 100), and the HbA1c value (notation value) of the calibration sample found ahead of time by reference method. If the calibration curve is produced using two or more calibration points, for example, multiple measurements are made for each of the two or more calibration points, and these are subjected to linear regression by the least square method, etc., to obtain a linear calibration curve. Also, an average may be found of the values obtained by each of the multiple measurements, and these connected together with a straight line to produce a calibration curve.

The data indicating the calibration curve can be recorded as the values of coefficients a and b in the linear equation y = ax + b indicating a calibration curve, for example. One of these values for a and b can also be prerecorded as a fixed value and used to calculate only the value of the other, and recorded being associated with the ID of the separation device. Thus, fixed values recorded ahead of time to the analysis device 1 or the separation device 4 can be used for parameters of part of the calibration data. It should be noted that the calibration data is not limited to being a value that indicates a calibration curve. For example, it may be a table or matrix that correlates the separation results with the calibration values.

The calibration unit 11 associates the calibration data obtained by separating the calibration sample with the separation device 4, with the ID of this separation device 4 and records it to the recording device 3 (S6). For instance, as shown in FIG. 3, calibration information (coefficients a and b of a formula indicating a calibration curve) is associated with the ID of a separation device, and the resulting data is recorded to the recording device 3.

The computing unit 12 reads the calibration data recorded to the recording device 3 by the calibration unit 11 (S7). The computing unit 12 also causes the separation device 4 to separate the measurement target component in the sample (the one to be measured) and causes the detector 2 to detect this, which yields measurement data for the sample. This measurement data and the calibration data that has been read are used to cover to a value expressing the amount of measurement target component in the sample. For example, when measuring HbA1c, the computing unit 12 can convert the HbA1c measurement result in a chromatogram (this is equal to the surface area of the HbA1c fraction divided by the surface area of the total Hb fraction, times 100) into a traceable HbA1c value by reference method using the above-mentioned calibration curve. Thus, data calibration can be executed using a calibration curve and sample measurement data. It should be noted that the sample measurement performed in S8 is not limited to a single iteration, and a plurality of samples may be measured continuously.

In the above embodiment, once calibration is performed with the analysis device 1 for the mounted separation device, there is no need for calibration to be performed again if the separation device is removed and then mounted again. This has the following effects, for example. First, the work entailed by calibration is eliminated, and the separation device can be replaced more often. Also, after a separation device is remounted, a sample can be measured more quickly Further, when measurement is complete, a delicate separation device can be removed from the analysis device for safe storage. In addition, fewer calibrations are needed, so the cost is reduced (such as a reduction in expanse for samples used for calibration).

### Second Embodiment

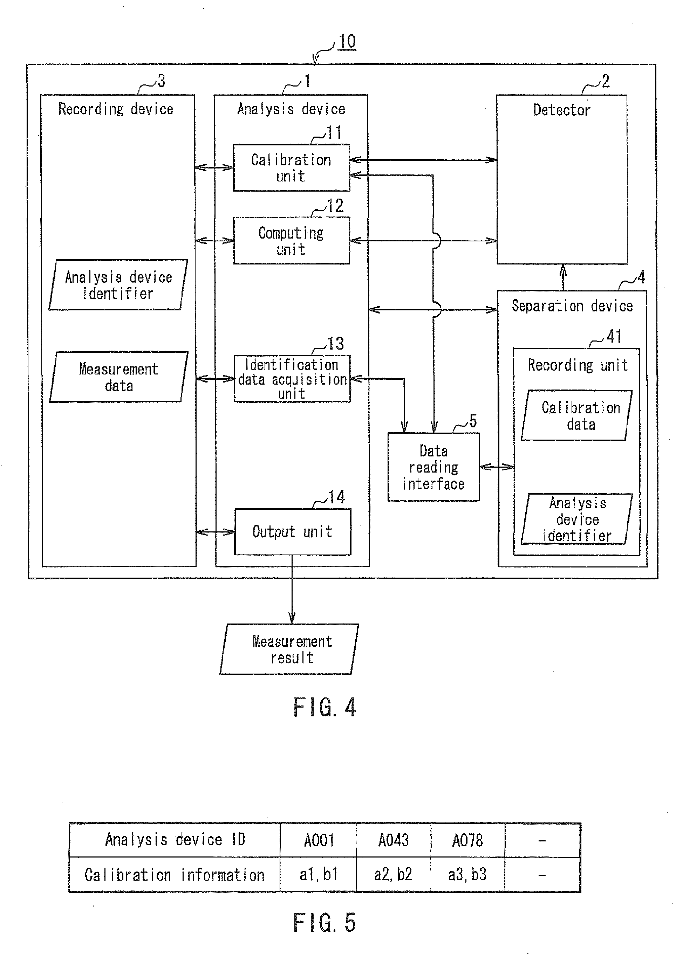

FIG. 4 is a function block diagram showing an example of the configuration of an analysis system that includes an analysis device in a second exemplary embodiment. In FIG. 4, function blocks that are the same as in FIG. 1 are numbered the same. In the example shown in FIG. 4, the calibration data is associated with the identifier of the analysis device and recorded to the recording unit 41 of the separation device 4. Specifically in this embodiment, calibration data and the ID of the analysis device 1 which has undergone calibration are recorded to the separation device 4. Consequently when the separation device 4 is mounted to the analysis device, the analysis device 1 can read from the recording unit 41 of the separation device 4 the calibration data corresponding to its own unique ID, and perform measurement.

For example, if the analysis system 10 is a capillary electrophoresis device, the separation device 4 can be a microchip. An EEPROM or other such recording device is disposed, for example, as the recording unit 41 (an example of a recording device) on the microchip. In this case, the data reading interface 5 can be an EEPROM controller. Thus, with this embodiment, the separation device comprises a recording device for recording calibration data. The recording device of the separation device is accessible from the analysis device in a state where it is mounted to the analysis device. In order words, it is not necessary for the recording device to be accessible from the analysis device even in a state where the separation device is not mounted to the analysis device 1.

When the separation device 4 is mounted, the calibration unit 11 accesses the recording unit 41 (EEPROM) of the separation device 4 via the data reading interface 5, and can determine whether or not calibration data corresponding to the ID of the analysis device 1 has been recorded in the EEPROM of the separation device 4. If there is no calibration data corresponding to the ID of the analysis device 1, calibration processing is executed by the calibration unit 11.

This calibration processing can be executed in the same manner as in the first embodiment above. When the calibration unit 11 produces calibration data for the separation device 4, it is associated with the ID of the analysis device 1 and recorded to the recording unit 41 of the separation device 4.

The computing unit 12 acquires calibration data corresponding to the ID of the analysis device 1 from the recording unit 41 of the separation device 4 via the data reading interface 5, and can use this to calculate the quantitative value of the measurement target component. Alternatively, the calibration unit 11 can record calibration data to the recording device 3 of the analysis device 1, and the computing unit 12 can use the calibration data of the recording device 3 to calculate the quantitative value of the measurement target component.

Furthermore, in this embodiment, the analysis device 1 can operate the same as in the operation example described through reference to FIG. 2 in the first embodiment above, for example. In this embodiment, in S3 in FIG. 2, the calibration unit 11 first reads the ID of the analysis device 1 itself, which is recorded to the recording device 3 of the analysis device 1, and then accesses the recording unit 41 of the separation device 4 to determine whether or not there is calibration data corresponding to the ID of the analysis device 1 that has been read (i.e., S4 in FIG. 2). If there is no calibration data corresponding to the ID of the analysis device (i.e., No in S4), the calibration unit 11 executes calibration processing (S5). Also, in S6, the calibration unit 11 associates the ID of the analysis device 1 and the ID of the separation device 4 with the calibration data, and records the result to the recording unit 41 of the separation device 4. FIG. 5 is a diagram showing an example of a separation device in which calibration data that has been recorded after being associated with the ID of the analysis device 1. In the example shown in FIG. 5, calibration information is recorded for every ID of the analysis device 1.

The same effect can be obtained with this second embodiment as in the first embodiment. Also, since the calibration data is recorded to the recording device of the separation device after being associated with the ID of a device that has undergone calibration, if the separation device is mounted to various kinds of analysis device, for example, the proper calibration data that matches the mounted analysis device can be provided to each analysis device. Also, a measurement device having a recording device for recording calibration data associated with the ID of the analysis device 1, such as the separation device of the second embodiment, is an embodiment consistent with the present disclosure.

### Third Embodiment

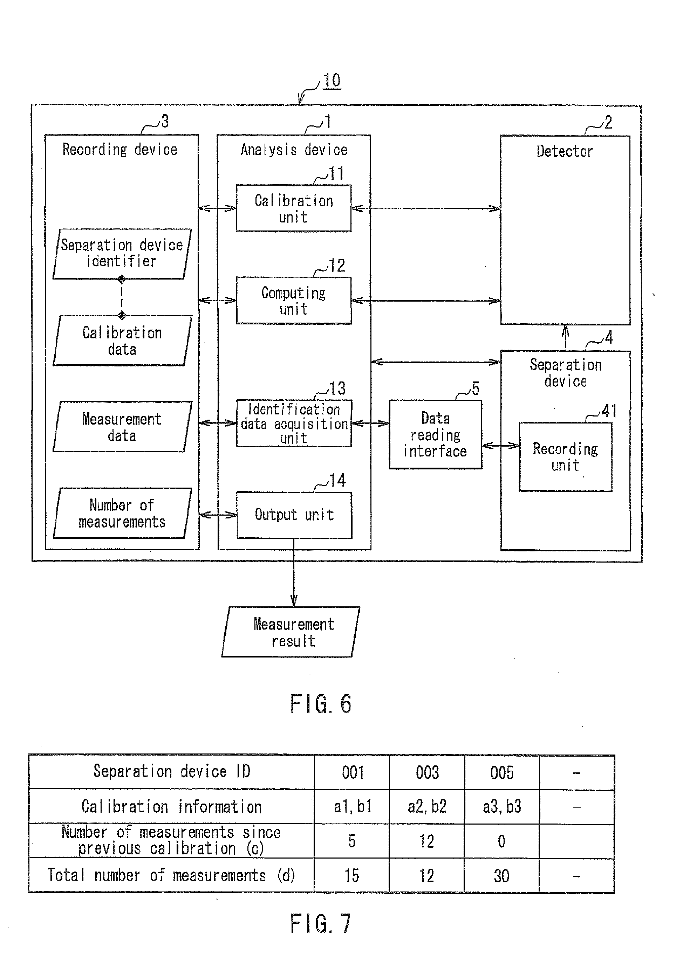

FIG. 6 is a function block diagram showing an example of the configuration of an analysis system that includes an analysis device in a third exemplary embodiment. In FIG. 6, function blocks that are the same as in FIG.1 are numbered the same. In the example shown in FIG. 6, the number of measurements is further recorded to the recording device 3. This embodiment differs from the first and second embodiments in that the number of times a separation device was used for measurement is recorded to the recording device 3 or the recording unit 41, and calibration and measurement are controlled on the basis of this.

In this embodiment, the computing unit 12 pre-stores the number of times a component has been measured using the separation device 4 in the recording device after associating this number with the ID of the separation device 4, and determines whether or not measurement is possible with this separation device based on this number of measurements.

Also, in addition to or instead of the functions of the above-mentioned computing unit, the computing unit 12 records the number of measurements of the separation device 4 after the previous calibration and/or the elapsed time since the previous calibration associated with the identifier of the separation device 4 to the recording device, and the calibration unit determines whether or not to perform calibration based on the elapsed time since the previous calibration and/or the number of measurements after the previous calibration.

For example, the number of measurement samples since the previous calibration and the total number of samples measured using the separation device 4 can be recorded as the number of measurements since the previous calibration and the total number of measurements of the separation device, respectively. These numbers of measurements can be associated with the separation device or the analysis device 1 and recorded to the recording device 3 or the recording unit 41. Also, the elapsed time since the previous calibration using the separation device 4 can be associated with the ID of the separation device or the ID of the analysis device 1 and recorded to the recording device 3 or the recording unit 41.

FIG. 7 is a table showing an example of data recording these numbers of measurements. In the example shown in FIG. 7, calibration information, the number of measurements c since the previous calibration, and the total number of measurements d are associated with each ID of the separation device, and this data can be recorded to the recording device 3. Also, calibration information, the number of measurements c since the previous calibration, and the total number of measurements d can be associated and recorded for each ID of the analysis device 1, in which case the data can be recorded to the recording unit 41. Also, for example, the elapsed time p since the previous calibration and the total elapsed time q since the first calibration may be recorded instead of or in addition to the number of measurements c and the total number of measurements d shown in FIG. 7.

Every time one sample is measured, the computing unit 12 can add "+1" to the total number of measurements (update the number of measurements recorded to the recording device). When the calibration unit 11 executes calibration, the number of measurements since the previous calibration recorded to the recording device is initialized to "0," after which "+1" is added to the number of measurements since the previous calibration every time the measurement of one specimen ends.

These values for the total number of measurements and the number of measurements since the previous calibration are preferably monitored constantly by the computer that corrects the separation device 4. The computing unit 12 can perform control such that when the total number of measurements reaches a specific value, for example, the use of that separation device is halted, and subsequent measurements are handled by another separation device. Also, when the value of the number of measurements since the previous calibration reaches a specific value, the calibration unit 11 can recommend that the operator correct again, or can automatic calibration processing may be executed.

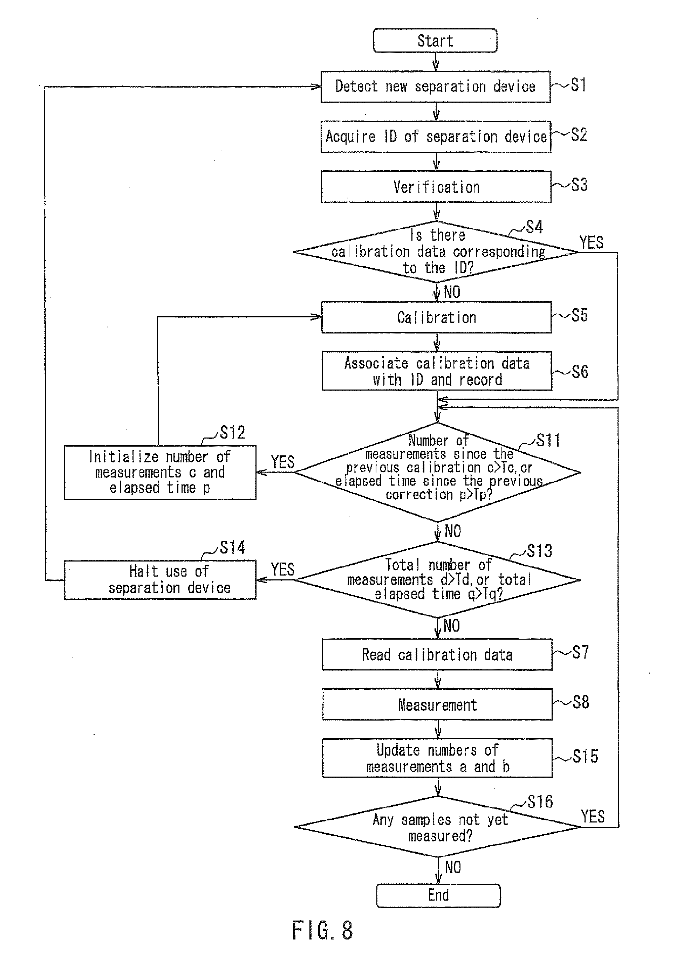

FIG. 8 is a flowchart showing an example of the operation of an analysis device in this embodiment. In FIG. 8, processing steps that are the same as the processing steps shown in FIG. 2 are numbered the same. In the example shown in FIG. 8, the computing unit 12 determines the number of measurements c and the elapsed time p since the previous calibration (S11) and determines the total number of measurements d and the total elapsed time q prior to the reading of calibration data (S7) and measurement (S8).

In S11, if the computing unit 12 determines that the number of measurements c since the previous calibration has exceeded a specific threshold Tc, or that the elapsed time p since the previous calibration has exceeded a specific threshold Tp, then the number of measurements c and the elapsed time p are initialized, that is, updated to "0" (S12), and the calibration unit 11 executes calibration processing (S5).

In S13, if the computing unit 12 determines that the total number of measurements d has exceeded a specific threshold Td, or that the total elapsed time q since the first calibration has exceeded a specific threshold Tq, then the use of that separation device is halted (S14), and a new separation device is selected (S1). In this embodiment, we will assume that a plurality of separation devices 4 can be mounted, or that the separation device 4 includes a plurality of capillaries. With this configuration, if the number of measurements for one separation device or capillary exceeds the upper limit, then measurement is performed using another separation device or capillary.

If the number of measurements c and the total number of measurements d since the previous calibration have neither reached the upper limit (No in S11, and No in S13), then the computing unit 12 executes calibration data reading (S7) and measurement (S8). When the measurement ends, "+1" is added to the numbers of measurements c and d (S15). The measurement and number of measurements determination processing (S7, S8, and S11 to S15) are repeated until there are no more samples that have yet to be measured (No in S16).

The operation shown in FIG. 8 and discussed above is just an example, and the operation of the analysis device is not limited to this. For instance, the determination of just S11 or S13 may be executed, and not the other. Also, as processing when the total number of measurements exceeds the upper limit, a warning message may be outputted to the operator instead of or in addition to the above-mentioned processing.

Also, in S11, in the above example, it was determined whether c > Tc or p > Tp was true or false, but it may instead be determined whether both c > Tc and p > Tp are true or false. Similarly, in S13, it may be determined whether both d > Td and q > Tq are true or false. Further, in S11 and S13, just the number of measurements or the elapsed time may be determined, and not the other. Specifically, it may be determined in S11 whether c > Tc is true or false, and in S13 whether d > Td is true or false, or it may be determined in S11 whether p > Tp is true or false, and in S13 whether q > Tq is true or false.

The additional functions in this embodiment, as discussed above, are particularly useful in the field of clinical testing, which demands high precision and high accuracy of measurement data. Examples of the practical application of a separating analysis method in the field of clinical testing include HbA1c and glycoalbumin, which are diabetes treatment markers, HbA2 and HbF, which are beta-thalassemia diagnostic markers, and HbS and HbC, which are abnormal hemoglobinemia markers. If these functions are applied to the measurement of these markers, it will be possible to obtain measurements with high accuracy and precision. Furthermore, the field of application of this embodiment is not limited to the clinical testing field. This embodiment can also be applied to the first and second embodiments above, as well as to the fourth and fifth exemplary embodiments given below.

### Fourth embodiment

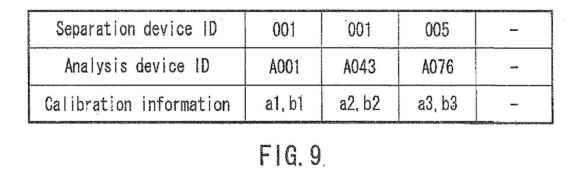

A fourth exemplary embodiment is a modification of the calibration data recording form. FIG. 9 is a table showing an example of calibration data in this embodiment. In the example shown in FIG. 9, calibration information is recorded for every combination of the ID of the analysis device 1 and the ID of the separation device 4. Associating and recording the ID of the analysis device 1 and the ID of the separation device 4 with the calibration data makes it possible to read the proper calibration data according to the combination of the ID of the analysis device 1 and the ID of the separation device 4. For instance, if the recording device 3 to which calibration data is recorded is a recording medium that can be accessed from a plurality of analysis devices, then each of those analysis devices can read the proper calibration data according to the combination of the analysis device and the separation device.

Since the measurement data changes with the combination of the analysis device and the separation device, if calibration data is recorded for all of these combinations, then once calibration is performed for a given combination, no calibration will be necessary when measurement is performed with the same combination again. This embodiment can also be applied to the first to third exemplary embodiments above, to the fifth exemplary embodiment given below, and to modifications of these.

### Fifth Embodiment

In a fifth exemplary embodiment, the separation device 4 comprises a plurality of channels for separating a sample. Accordingly the ID of the separation device 4 includes ID's for the various channels in order to identify each of the channels provided to the separation device. The calibration unit 11 produces or updates the calibration data for every channel, associates the ID for the channels with each set of calibration data, and records the result. The computing unit 12 acquires measurement data obtained by detection in one channel for the measurement target component by the detector 2. The computing unit 12 then acquires from the recording device 3 or the recording unit 41 the calibration data associated with the ID for this one channel, which is included in the ID for the separation device 4 acquired by the identification data acquisition unit 13. This calibration data and measurement data are used to calculate a value indicating the amount of the measurement target component. Consequently, when a separation device having a plurality of channels is connected, the proper calibration data can be recorded ahead of time for every channel.

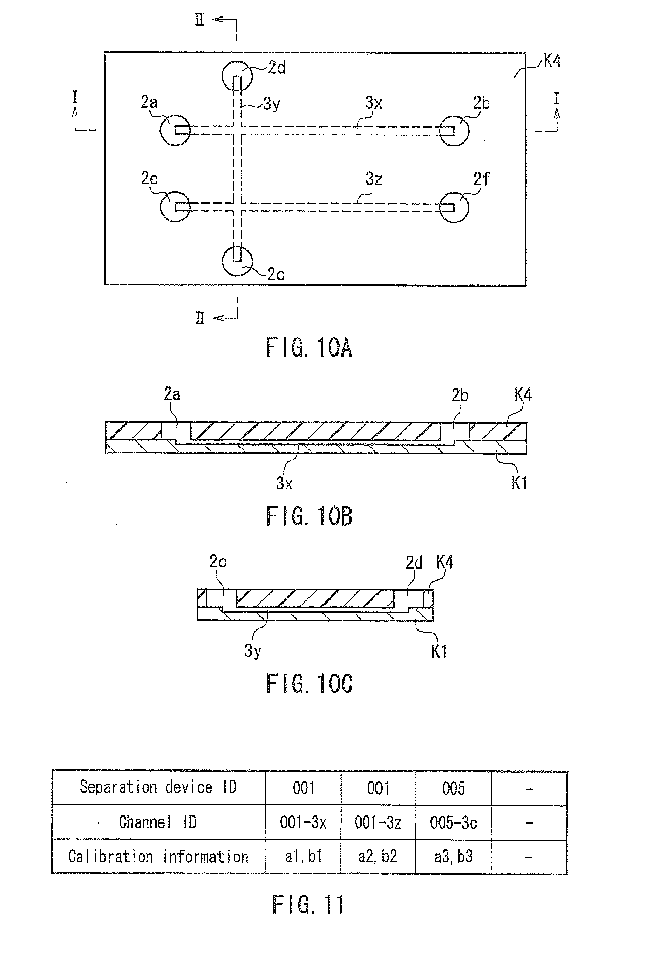

FIGS. 10A to 10C are diagrams showing an example of the configuration of a separation device that has been made into a microchip (hereinafter referred to as an analysis chip) in this embodiment. FIGS. 10A to 10C show an example of the separation device of an analysis device that makes use of capillary electrophoresis. FIG. 10A is a plan view of the analysis chip in this example, FIG. 10B is a cross section along the I-I line in FIG. 10A and FIG. 10C is a cross section along the II-II line in FIG. 10A. In these drawings, the size, proportions, and so forth of the various constituent elements may not be the same as in actuality in order to make them easier to understand.

In the example shown in FIGS. 10A to 10C, an upper substrate K4 is laminated over a lower substrate K1 to create an analysis chip. A plurality of (six in this example) through-holes are formed in the upper substrate K4. The bottoms of the six through-holes formed in the upper substrate K4 are sealed off by the lower substrate K1, which forms six liquid reservoirs 2a to 2f. Two parallel grooves and a groove that intersects these at a right angle are formed in the lower substrate K1. The upper parts of the grooves formed in the lower substrate K1 are sealed off by the upper substrate K4, which forms capillary channels 3x and 3z used for sample analysis, and a capillary channel 3y used for sample introduction. The six liquid reservoirs 2a to 2f include a first introduction reservoir 2a, a first recovery reservoir 2b, a second introduction reservoir 2c, a second recovery reservoir 2d, a third introduction reservoir 2e, and a third recovery reservoir 2f. The first introduction reservoir 2a and the first recovery reservoir 2b communicate with each other through the capillary channel 3x used for sample analysis. The second introduction reservoir 2c and the second recovery reservoir 2d communicate with each other through the capillary channel 3y used for sample introduction. The third introduction reservoir 2e and the third recovery reservoir 2f communicate with each other through the capillary channel 3z used for sample introduction. The capillary channel 3z and the capillary channel 3x are disposed in parallel, both intersect the capillary channel 3y used for sample introduction, and communicate with each other through the portion where they intersect the capillary channel 3y.

Although not shown in the drawings, electrodes for capillary electrophoresis may also be provided to the analysis chip. For example, six electrodes can be disposed so that one end of each is disposed in the liquid reservoirs 2a to 2f.

Also, a liquid supply unit for supplying electrophoretic fluid and sample to the analysis chip, and a voltage applicator for applying voltage to the capillary channels may be provided. Also, part of each of the capillary channels 3y and 3z may be the measurement site, and the detector 2 can be disposed so that the detection site of the detector 2 is located at these measurement sites. When the analysis chip thus has a plurality of measurement sites, at least the measurement sites during voltage application are preferably constituted so as to be mutually independent channels.

The analysis conditions for the sample that is supplied, the applied voltage, the flux of liquid, and so forth in the capillary channels 3y and 3z can be controlled, for example, by control signals from the analysis device 1. For instance, the calibration unit 11 also acquires the ID of the capillary channels, in addition to the analysis chip, when an analysis chip having a plurality of capillary channels is mounted. The ID of the capillary channels may, for example, be acquired along with the ID of the analysis chip acquired by the identification data acquisition unit 13, or may be acquired by a different route from that of acquisition by the identification data acquisition unit 13. The calibration unit 11 can determine whether or not to produce calibration data for every capillary channel. For example, calibration data can be produced when there is no calibration data in the recording device 3 that corresponds to the ID of the capillary channels.

The computing unit 12 controls measurement by the capillary channels of the analysis chip, and obtains measurement data by separating the sample and detecting the measurement target component for every capillary channel. When measurement data is obtained for a given capillary channel, calibration data corresponding to the ID of that capillary channel is read from the recording device 3, and this calibration data can be used to correct the measurement data.

FIG. 11 is a table showing an example of calibration data recorded to the recording device 3. In the example shown in FIG. 11, calibration information is recorded for every combination of separation device ID and channel ID. Thus, the proper calibration data can be recorded for every channel by associating channel ID with calibration information and recording it, along with separation device ID.

In the first to fifth exemplary embodiments above, the description was of the measurement of hemoglobin, and particularly the HbA1c value, but the analyte in the present disclosure is not limited to the example given above. For instance, the following analytes can also be measured using the analysis device described in any of the first to fifth exemplary embodiments given above. The "analyte" can be, for example, a protein, an in vivo substance, a substance in the blood, or the like, which are examples of a biologically derived component. Specific examples of proteins include hemoglobin, albumin, and globulin. Examples of hemoglobin include glycated hemoglobin, variant hemoglobin, modified hemoglobin, and other hemoglobin minor components. More specifically, examples include hemoglobin A0 (HbA0), hemoglobin A1c (HbA1c), hemoglobin A2 (HbA2), hemoglobin S (HbS, sickle cell hemoglobin), hemoglobin F (HbF, fetal hemoglobin), hemoglobin M (HbM), hemoglobin C (HbC), methemoglobin, carbamylated hemoglobin, and acetylated hemoglobin. HbA1c comes as stable HbA1c and unstable HbA1c. HbA1c refers to one in which glucose is bonded to the N terminal of a hemoglobin β chain, and preferably refers to stable HbA1c (s-HbA1c). Examples of albumin include glycoalbumin and nonglycoalbumin. Specific examples of in vivo substances, substances in the blood, and so forth include bilirubin, hormones, and metabolic substances. Examples of hormones include a thyroid stimulation hormone, an adrenocorticotropic hormone, chorionic gonadotropin, insulin, glucagon, an adrenomedullary hormone, epinephrine, norepinephrine, androgen, estrogen, progesterone, aldosterone, and cortisol.

Also, the term "sample" is not limited to the above examples. The term "sample" in the above embodiment refers to a sample prepared from a sample raw material. Examples of the sample raw material include a biological sample including a biologically derived component, which preferably includes the above-mentioned analyte, and more preferably a sample containing hemoglobin. Examples of biological samples include blood, products derived from blood and containing a red blood cell component, saliva, and cerebrospinal fluid. Examples of blood include blood collected from an organism, preferably animal blood, more preferably mammal blood, and still more preferably human blood. Examples of products derived from blood and containing a red blood cell component include those separated or prepared from blood and containing a red blood cell component, examples of which include a blood cell fraction from which plasma has been removed, a blood cell condensate, freeze-dried blood or blood cells, a hemolyzed sample prepared by hemolyzing whole blood, centrifuged blood, and spontaneously-sedimented blood.

Also, the measurement of the HbA1c value was described in the first to fifth embodiments above, but the amounts and values for other glycated proteins can also be measured similarly. The term "glycated protein amount" here refers to the proportion of glycated protein in a sample or correction substance, and a specific example is the glycated protein concentration. Examples of glycated protein include glycated hemoglobin, hemoglobin A1c (HbA1c), glycated albumin, and glycated globulin.

The term "glycated protein value" can be a value expressing the ratio of glycated protein to the protein in question, for example, and indicates the glycation ratio of the protein being measured. Examples of the glycated protein value include the ratio of HbA1c versus hemoglobin (HbA1c concentration / hemoglobin concentration; units: mmol/mol or %), and the ratio of glycated albumin versus albumin (glycated albumin concentration / albumin concentration).

Also, in the first to fifth exemplary embodiments above, the description was of a situation in which the measurement device that holds the sample was a separation device, but embodiments consistent with the present disclosure can also be applied to measurement devices and analysis devices that make use of some analysis method other than a separating analysis method. For example, a sensor corresponding to a blood glucose measurement meter, a biochemical measurement tool, a chip for a gene detection device, a chip for a measurement device that makes use of a fluorescence method, or the like can be used as a measurement device. Examples of measurement devices that make use of some analysis method other than a separating analysis method will now be described. The following examples can be applied to one or more of the first to fifth exemplary embodiments above.

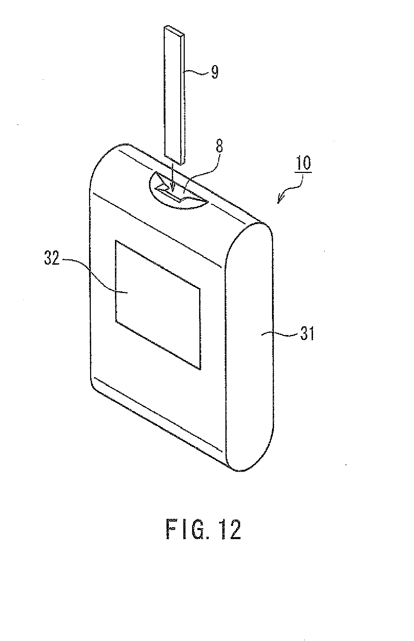

FIG. 12 is an oblique view of an example of a portable treatment device that can be applied in embodiments according to the present disclosure. In the example shown in FIG. 12, a treatment device 10 is used to measure the condition of an organism, examples of which include a blood glucose meter, a sphygmomanometer, a lactic acid meter, a ketone body measurement device, a thermometer, a urine test paper meter, and a lipid measurement device. An analysis device 1 and a detector 2 that match the application are housed inside the main body 31 of the treatment device 10. The treatment device 10 can be formed in a size that fits in the palm of the hand, and can be carried around by the user, such as a patient, a physician, or a nurse.

The treatment device 10 may be used as a portable blood glucose meter for measuring the glucose level of a patient's blood. In this case, the patient's blood is provided by a sensor 9, and the main body 31 comprises a sensor inlet 8 for inserting the flat sensor 9. The sensor 9 has a reagent in its interior, and blood reacts with the reagent inside the sensor 9. The sensor 9 here is an example of the measurement device 4. The analysis device 1 and the detector 2 inside the main body 31 have the function of measuring the blood glucose level of blood that has reacted with the reagent, by colorimetry or an electrochemical method. Measurement with the measurement device begins right away when the sensor 9 is inserted into the sensor inlet 8. The measurement result is displayed on a display screen 32 provided to the main body 31.

An electronic chip (an 1C tag, a memory, etc.), printing (a barcode, etc.), engraving, or the like is provided to the sensor 9 as a recording unit 41. In a state in which the sensor 9 has been inserted into the main body 31, the analysis device inside the main body 31 can read information about the recording unit 41. An identifier (ID) for the sensor 9 can be recorded to the recording unit 41 of the sensor 9. The identifier of the sensor 9 may be information that identifies the type of sensor, for example. Also, a set of calibration data and an identifier of the analysis device 1 may be recorded instead of the identifier of the sensor 9.

The detector 2 inside the main body 31 detects the color of blood that has reacted with the reagent in the sensor 9, and the analysis device 1 can calculate the blood glucose level from the detection value produced by the detector 2. Here, just as in the above embodiment, an identifier of the sensor 9 is acquired from the recording unit 41 of the sensor 9, and calibration data corresponding to the identifier of the sensor 9 can be used to convert the detection value into a blood glucose level. Alternatively, the analysis device 1 can use calibration data corresponding to an identifies of the analysis device 1 recorded to the recording unit 41 of the sensor 9, to calculate a blood glucose level. This allows the blood glucose level to be calculated using the proper calibration data for each sensor 9 or for each type of sensor 9.

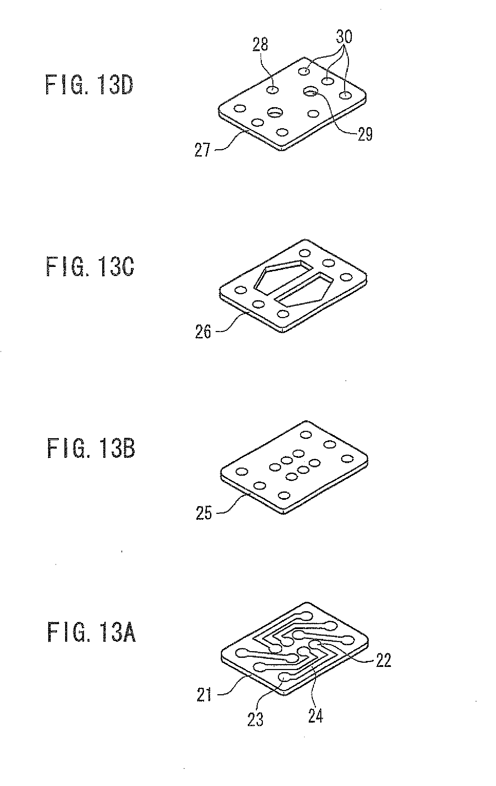

FIG. 13 is an exploded oblique view of an example of an ion activity measurement tool that can be applied to embodiments consistent with the present disclosure. With the ion activity measurement tool shown in FIG. 13, an electrode metal layer (a) consisting of an electrode part 22, a terminal part 23, and a conductive wire part 24, a first resist film 25 (b), and an ion selective film are formed on a substrate 21. Consequently, this is made up of three pairs of electrodes. A second resist film 26 (c) and a cover board 27 (d) are affixed to the substrate 21. The cover board 27 can be formed from a film of an insulating material provided with a sample supply hole 28, an air vent hole 29, and a terminal exposure hole 30. The first resist film 25, the cover board 27, and the second resist film 26 form a liquid reservoir.

This ion activity measurement tool has at least one pair of electrodes, and is designed so that one of the electrodes comes into contact with the liquid sample, and the other comes into contact with a reference liquid. The electrode in contact with the liquid sample usually has ion selectivity. The potential difference corresponding to the difference in the ion activity produced between the electrodes when the liquid sample and the reference liquid each come into contact with the electrodes is measured, and this potential difference is converted into concentration. The ion activity measurement tool is an example of a measurement device.

The detector 2 can be a voltage meter that measures the above-mentioned electromotive force, for example. The analysis device 1 uses the above-mentioned calibration curve as calibration data, and calculates the concentration from the electromotive force.

An electronic chip (an IC tag, a memory, etc.), printing (a barcode, etc.), engraving, or the like is provided to the ion activity measurement tool as the recording unit 41, for example. One or more of the first to fifth embodiments given above can be applied for the operation and function of the detector 2 and the analysis device 1 and the data of the recording unit 41. Consequently, the calibration data used to convert the measured potential difference into a concentration can be appropriately set according to the ion activity measurement tool.

In addition, the first to fifth exemplary embodiments above can be applied to any analysis device that uses a removable measurement device to analyze components contained in a sample. This will be apparent to a person skilled in the art, from the features of the present disclosure.

Embodiments consistent with the present disclosure can be utilized or used for measurement precision of an analysis device in fields such as medical devices, clinical testing devices, and so on.

The exemplary embodiments disclosed in this application are to be considered in all respects as illustrative and not limiting may be embodied in other forms without departing from the spirit or essential characteristics thereof. The scope of the invention is indicated by the appended claims rather than by the forgoing description, and all changes which come within the meaning and range of equivalency of the claims are intended to be embraced therein.

## Claims

1. An analysis device that controls a detector for detecting a measurement target component in the sample held by a removable measurement device for holding the sample in a state that allows measurement, and thereby acquires measurement data and analyzes the measurement target component, the analysis device comprising:
an identification data acquisition unit adapted to acquire an identifier of the measurement device mounted to the analysis device;
a calibration unit adapted to record calibration data associated with the identifier of the measurement device to at least one of recording device provided to the analysis device or recording device that is accessible from the analysis device; and
a computing unit adapted to calculate a value indicating information related to the measurement target component by using measurement data obtained by detection of the measurement target component by the detector and calibration data that has been recorded to the recording device associated with the identifier of the measurement device acquired by the identification data acquisition unit.

2. The analysis device according to claim 1, wherein the calibration unit is adapted to determine whether or not calibration data associated with the identifier of the mounted measurement device has been recorded to the recording device when the measurement device is mounted, and if the calibration data has not been recorded to the recording device, the calibration data is produced and recorded to the recording device with being associated with the identifier of the mounted measurement device.

3. The analysis device according to claim 1 or 2,
wherein the measurement device has recording device which can be accessed from the analysis device when the measurement device is mounted to the analysis device;
the identification data acquisition unit acquires an identifier of an analysis device,
the calibration unit records the identifier of the analysis device associated with the calibration data to the recording device provided to the mounted measurement device, and
the computing unit calculates a value indicating the information related to the measurement target component by using measurement data obtained by detection of the measurement target component by the detector and calibration data that has been recorded to the recording device of the measurement device associated with the identifier of the analysis device acquired by the identification data acquisition unit.

4. The analysis device according to any of claims 1 to 3, wherein the calibration unit is adapted to determine whether or not the calibration data associated with the identifier of the analysis device has been recorded to the recording device of the measurement device when the measurement device is mounted, and if the calibration data has not been recorded to the recording device, the calibration data is produced and recorded to the recording device with being associated with the identifier of the analysis device.

5. The analysis device according to any one of claims 1 to 4, wherein the computing unit is adapted to record the number of measurements of the measurement target component using the measurement device associated with the identifier of the measurement device to the recording device, and to determine whether or not measurement is performed using the measurement device based on the number of measurements.

6. The analysis device according to any one of claims 1 to 4, wherein the computing unit is adapted to record the elapsed time and/or the number of measurements after the previous calibration of the measurement device associated with the identifier of the measurement device to the recording device, and the calibration unit is adapted to determine whether or not to perform calibration based on the elapsed time and/or the number of measurements after the previous calibration.

7. The analysis device according to any one of claims 1 to 6, wherein the calibration unit is adapted to record the calibration data associated with the identifier of the analysis device and the identifier of the measurement device, and to determine whether or not calibration data associated with the identifier of the mounted measurement device and the identifier of the analysis device has been recorded to the recording device when the measurement device is mounted, and if the calibration data has not be recorded to the recording device, the calibration data is produced and recorded to the recording device with being associated with the identifier of the mounted measurement device and the identifier of the analysis device.

8. The analysis device according to any one of claims 1 to 7,
wherein the measurement device comprises a plurality of channels,
the identifier of the measurement device includes a channel identifier for each of the plurality of channels,
the calibration unit is adapted to produce or update the calibration data for each of the channels, and to record the channel identifier associated with the calibration data, and
the computing unit is adapted to calculate a value indicating information related to the measurement target component by using the measurement data obtained by detection of the measurement target component at one channel by the detector and calibration data that has been recorded to the recording device associated with the channel identifier of the one channel included in the identifier of the measurement device acquired by the identification data acquisition unit.

9. The analysis device according to any one of claims 1 to 8, wherein the calibration unit is adapted to produce or update the calibration data by using measurement data obtained by detection of the measurement target component in a calibration sample in the measurement device, and to record the calibration data associate with the measurement device identifier.

10. A measurement device that is attachable to and removable from an analysis device and is a device for holding a sample in a state that allows measurement comprising,
recording device for recording calibration data that is associated with an identifier of the analysis device and is used in calculating a measurement target component from measurement data obtained by detection of the measurement target component in the sample held by the measurement device.

11. An analysis program causing a computer to execute processing that controls a detector for detecting a measurement target component in the sample held by a removable measurement device for holding the sample in a state that allows measurement, and thereby acquires measurement data and analyzes the measurement target component, the analysis program causing the computer to execute following processes:
identification data acquisition processing of acquiring an identifier of the measurement device mounted to the analysis device;
calibration processing of recording calibration data associated with the identifier of the measurement to recording device; and
computation processing of calculating a value indicating information related to the measurement target component by using measurement data obtained by detection of the measurement target component by the detector and calibration data that has been recorded to the recording device associated with the identifier of the measurement device acquired by the identification data acquisition processing.

12. An analysis method executed by a computer that controls a detector for detecting a measurement target component in the sample held by a removable measurement device for holding the sample in a state that allows measurement, and thereby acquires measurement data and analyzes the measurement target component, the analysis method comprising:
an identification data acquisition step of acquiring an identifier of the measurement device mounted to the analysis device;
a calibration step of recording calibration data associated with the identifier of the measurement device to recording device; and
a computation step of calculating a value indicating information related to the measurement target component by using measurement data obtained by detection of the measurement target component by the detector and calibration data that has been recorded to the recording device associated with the identifier of the measurement device acquired by the identification data acquisition step.
